# EUROPEAN PATENT SPECIFICATION

(11) **EP 1 917 919 B1**
(45) Date of publication and mention of the grant of the patent: **25.09.2019**
(21) Application number: 07122916.5
(22) Date of filing: 13.08.2003
(51) Int. Cl.: A61B 17/08, A61B 17/064

(54) **Cardiac devices for percutaneous repair of atrioventricular valves**
Kardiovorrichtungen zur perkutanen Reparatur atrioventrikulärer Klappen
Dispositifs cardiaques de réparation percutanée des valvules auriculoventriculaires

(30) Priority: 13.08.2002 US 403073 P
(43) Date of publication of application: 07.05.2008
(62) Divisional of application: 03785293.6
(73) Proprietor: Levine, Robert, Brookline, MA 02146-5246 (US)
(72) Inventor: Levine, Robert, Brookline, MA 02146-5246 (US)
(74) Representative: Hansen, Norbert

(56) References cited:
- WO-A-00/60995
- US-A- 4 543 090
- US-A- 5 458 573
- US-B1- 6 178 346
- US-B1- 6 323 459

## Description

### Field of the Invention

The present invention relates generally to devices (i.e., articles of manufacture, apparatus, systems, instruments) for treating heart disease and, in particular, to devices for minimally invasive repair of atrioventricular valve regurgitation (MR) occurring in the context of mitral valve prolapse (MVP), flail mitral valve, and/or ischemic MR.

### Background of the Invention

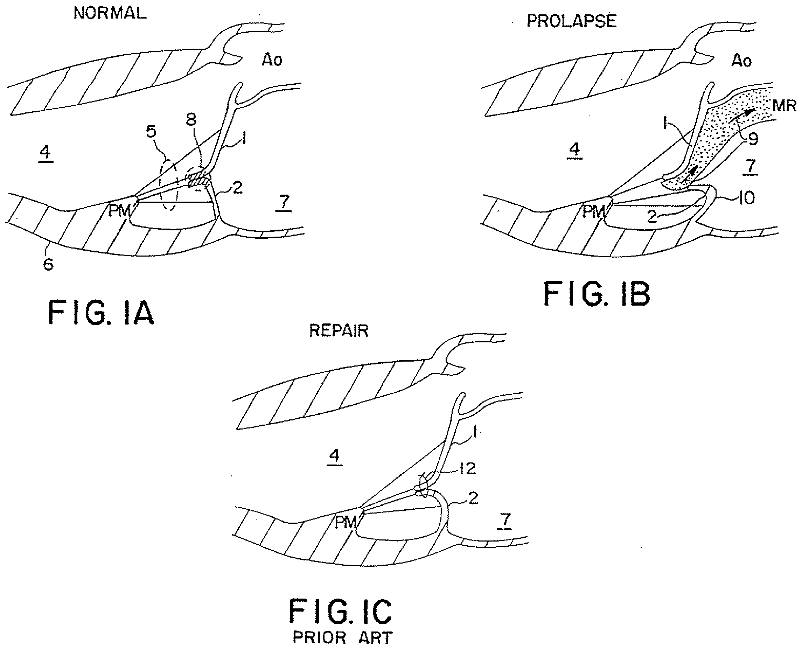

A mitral valve, such as shown in Figure 1A, including an anterior leaflet 1 and a posterior leaflet 2, is the inlet valve to the main heart pumping chamber (left ventricle 4). The mitral valve is forced to close when the ventricle 4 contracts, preventing backward flow of blood. To ensure that this valve does not prolapse backward (into left atrium 7) when the heart contracts, leaflets 1,2 are restrained by a network of tendinous chords 5 anchored to the posterior wall of the heart 6. The leaflets normally meet or coapt so that their tips are juxtaposed, along with up to one-third of their lengths, forming an effective seal 8 to prevent MR. In a variety of diseases, however, the leaflets fail to meet properly due to leaflet elongation or elongation or rupture of the chords 5. As shown in Figure 1B, one or more leaflet portions 10 may then prolapse into the left atrium 7, reducing coaptation and creating a gap between the leaflets 1,2 that allows MR to occur (blood flow illustrated by arrows 9.). Such regurgitation can produce heart failure, rhythm disorders, sudden death, and a predisposition to lethal heart valve infections. Therapy has until now required open-heart surgery to replace the valve or repair it by approximating leaflet tips.

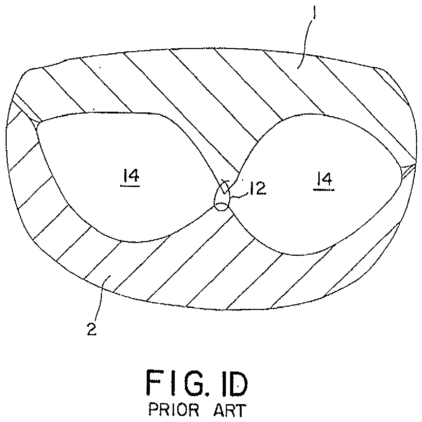

Alfieri, et. al, have described a possibly minimally invasive repair mechanism. As shown in Figure 1C, a suture 12 may be placed between the tips of the leaflets 1,2 to prevent prolapse of one leaflet relative to the other. As shown in Figure 1D, this produces a competent valve with two openings 14, one on each side of the suture 12 position. Such an orifice does not materially obstruct inflow of blood into the left ventricle.

Several approaches have been proposed to practice this repair in a minimally invasive manner. Specifically, Grimes (U.S. patent 6,312,447) teaches that a catheter, advanced across the interatrial septum into the left atrium, can grasp the leaflet tips using a suction apparatus at the end of the catheter. A fastener such as a staple or shape memory rivet is then inserted into the leaflet tips to effect the edge-to-edge closure repair.

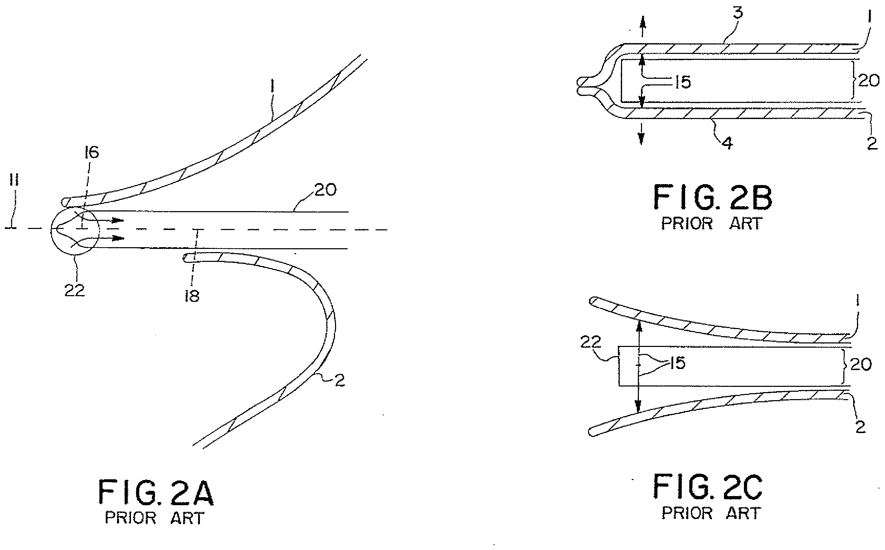

Previous approaches employing suctioning and/or suturing of leaflet tips suffer from a number of common limitations. First and foremost, as shown in Figure 2A, in patients with sufficient MR to warrant such procedure, the leaflets 1,2 are initially misaligned, limiting or precluding the ability of a single device 22 alone at the tip of a catheter 20, or multiple devices at a single location along the catheter center axis 11, to bring the leaflet tips, at spatially distinct positions 16 and 18, into juxtaposition in order to suture or fasten them together. Second, in order to be effective, a suction device for grabbing the leaflet tips must withdraw blood extremely rapidly. Unless blood is reinfused immediately, this can cause hypotension. Third, as illustrated in Figure 2B, unless an additional mechanism is inserted via the aorta to stabilize leaflet surfaces 3 and 4 that are not adjacent to the suction device, a rivet or staple 15 emerging from the catheter 20 may simply displace the leaflets 1,2 from the catheter tip 22 (as shown in Figure 2C) rather than successfully penetrate the leaflets. Fourth, approaches that employ grasping the leaflets generally do so in a plane perpendicular to the catheter, and do not provide a sufficiently large and stable leaflet surface area through which a staple or a suture can be inserted.

WO 00/60995 relates to cardiac valve repair. Regurgitation of an atrioventricular valve, particularly a mitral valve, can be repaired by modifying a tissue structure selected from the valve leaflets, the valve annulus, the valve chordae and the papillary muscles. These structures may be modified by suturing, stapling, snaring or shortening, using interventional tools which are introduced to a heart chamber. The tissue structures are temporarily modified prior to permanent modification. For example, apposed valve leaflets may be temporarily grasped and held into position prior to permanent attachment.

Thus, what is needed is a percutaneous mitral (or tricuspid) valve repair system that can overcome these and other limitations of the prior art. A single device accomplishing all of the above objectives and others is highly desirable.

### Summary of the Invention

The present invention provides novel devices and to treat heart disease related to mitral valve regurgitation occurring in the context of cardiac valve prolapse. The present invention allows for independent capture and repositioning of mitral leaflets regardless of whether they are aligned, and provides a stabilized large leaflet surface area through which sutures, staples, rivets and the like may be inserted. Use of a novel percutaneous catheter and other novel devices disclosed herein enables therapeutic maneuvers that do not require opening the chest or heart, however the present invention is not limited to percutaneous approaches. In the description that follows, the several specific preferred embodiments of the present invention referring to the invention's applicability to the mitral valve are meant in no way to be limiting. It will be readily appreciated that the present invention may additionally be effectively applied to leaflets of the tricuspid valve.

In a first aspect, the present invention provides a device to repair a mitral valve, comprised of a catheter assembly for delivering in a minimally invasive manner a pair of maneuverable grasping elements that are independently translatable through the catheter assembly to a position near the mitral valve, The grasping elements are reversibly and radially extendable from the catheter assembly so as to capture and stabilize portions of two valve leaflets in an apposed position so that a fastening mechanism (e.g., a staple, rivet or the like) which is also translated through the catheter assembly may be used to affix portions of the anterior and posterior leaflets to one another.

Shape memory materials, such as nickel titanium (e.g., NITINOL www.nitinol.com) may be used to manufacture several components of the present invention, including the curved distal end of the catheter assembly. The fastening mechanism are optionally composed of shape memory material as well.

The catheter assembly is formed of a pair of slidably linked catheters, each of which is responsible for delivering a grasping element to the mitral valve region.

The fastening mechanism is radially extendable and disengagable from each catheter through at least one side port of the catheter assembly. Described below is a mechanism by which the fastening mechanism may be disengaged from the catheter assembly after insertion through the leaflets, which occurs, significantly, without displacing the captured leaflets in a direction away from the catheter assembly and each other.

The grasping elements extend from the distal end of each catheter with reverse deformation so as to clip the ventricular side of the valve leaflets. In an alternative, but not mutually exclusive embodiment, the grasping elements extend from side ports in the catheter assembly and are steered so as to be able to grasp the atrial sides of the leaflets. In a preferred embodiment, two pairs of grasping elements are employed to grasp both sides of both the posterior and anterior leaflets. Various embodiments of the grasping elements are described below, including clip-like configurations and embodiments having operable jaws disposed at the ends of each grasping element.

In certain embodiments, the operation of each grasping element is triggered by sensors for detecting contact and/or proximity of the leaflet to be grasped to the grasping element.

External or internal cardiac imaging of the region of the mitral valve may be employed to facilitate proper positioning of the grasping elements and/or monitoring of effectiveness of the procedure. This imaging can alternatively be achieved through the use of ultrasound, magnetic resonance or fiber optics.

For a better understanding of the present invention, reference is made to the accompanying drawing and detailed description. The scope of the present invention will be pointed out in the appended claims.

### Brief Description of The Drawings

The advantages of the present invention will be apparent in the following detailed description of the illustrative embodiments thereof, which is to be read in connection with the accompanying drawing, wherein:
**Figures 1A-1C** are illustrations of a portion of a human heart showing normal, prolapsed and repaired mitral leaflet closure, diagrammed in a long-axis view of the left ventricle, as would be viewable by ultrasound imaging;
**Figure 1D** is an illustration of the mitral leaflets after repair, as viewable from the left atrial perspective with the valve open when the heart relaxes;
**Figure 2A** is a side view illustration of a cardiac valve demonstrating a limitation of a prior art catheter suctioning approach to grasping leaflets at different positions along a catheter due to prolapse;
**Figures 2B-2C** are side view illustrations of a cardiac valve demonstrating the displacement of mitral leaflets encountered by prior art approaches to valve stapling;
**Figures 3A-3B** are side view illustrations of a cardiac leaflet being grasped on its ventricular surface by a grasping element ;
**Figure 3C** is an illustration of a grasping element showing two side-arms for stabilizing a portion of the leaflet surface so that a staple or rivet can be inserted therebetween, as viewed from above, looking down onto the upper surface of the anterior mitral leaflet;
**Figures 3D-3E** are side view illustrations of a catheter assembly in accordance with the invention comprised of two linked catheters that independently translate grasped leaflets into apposition for fastening;
**Figure 4A** is a side view illustration of a mechanism for grasping the atrial surface of the leaflets, with independent translation of two grabbing elements to approximate the leaflets;
**Figures 4B,4C** are schematic illustrations of serrated and smooth pincer-like grabbing tools disposed at the end of certain embodiments of the grasping elements;
**Figure 5A** is a schematic illustration of a mechanism for inserting staples or rivets through an orifice in the catheter to fasten leaflets that have been approximated into apposition;
**Figures 5B-5E** are illustrations of operational steps for using a mechanism to disengage a staple from the catheter delivering it to the leaflet vicinity;
**Figure 6A** is an illustration of a retrograde catheter insertion with grasping elements grasping the ventricular surfaces of the leaflets;
**Figure 6B** is an illustration of a retrograde catheter insertion with grasping elements grasping both atrial and ventricular surfaces of the leaflets under imaging guidance;
**Figures 7A-7B** are illustrations of embodiments of catheter stabilization mechanisms.

### Detailed Description of Preferred Embodiments of the Invention

Preferred embodiments of the present invention will now be described with reference to the several figures of the drawing.

Numerous embodiments of the present invention will now be described in the context of mitral valve prolapse. Devices in accordance with the present invention are characterized in that they permit the anterior and posterior leaflets to be separately grasped and independently translated along an axis of a catheter assembly until leaflet apposition is achieved for the purpose of fastening the leaflets to one another. Use of the devices is not, however, limited to the mitral valve, as use on the tricuspid or any other cardiac valve will be readily appreciated by artisans.

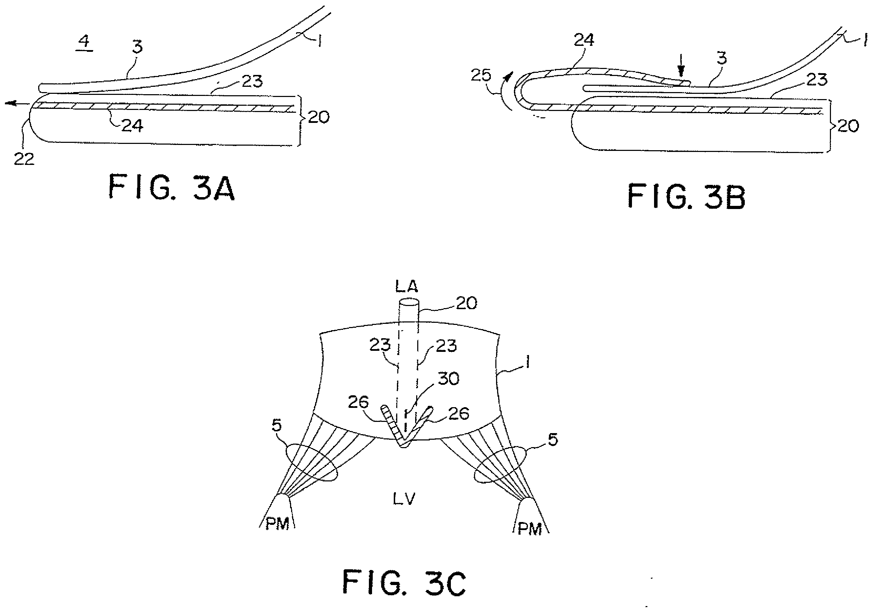

With reference to **Figure 3A****,** in one example not part of the invention a percutaneous catheter **20** is inserted through the interatrial septum into the left atrium **7** and then between the mitral leaflets (only anterior leaflet **1** shown) to the left ventricle **4** to a position adjacent leaflet **1** that is to be repositioned. Through catheter **20** at least one leaflet grasping element **24** can be reversibly extended from the catheter tip **22.** The grasping element **24** is composed of a shape memory material that has been pre-formed to have a shape and dimensions appropriate for clipping or compressing leaflet **1** against the external surface **23** of the catheter **20,** such as shown in **Figure 3B****.** When the grasping element **24** is extruded from the tip **22,** the element takes its pre-formed shape, which as depicted can be a reverse curve **25** that results in a clip-like structure on the ventricular surface **3** of leaflet **1.** The manufacturing of biocompatible shape memory components is well described in the art, thus will not be recited here.

**Figure 3C** depicts a view of the mitral valve looking down onto the upper surface of the anterior mitral leaflet. Note that the device operates in this case in the gap between the chordal connections **5** to each side of the leaflet. In an example, the grasping element **24** has preferably two side arms **26** that extend to either side of the catheter axis in a V-shaped or similar configuration having a separation there between sufficient to allow a staple or rivet **30** to be inserted through the leaflet unopposed by the grasping element. This design provides greater stability than a single point of leaflet contact, and allows a staple or rivet **30** to be inserted between the side arms **26** of the grasping element without the leaflet being displaced in the process. The design also provides a relatively large, stabilized leaflet surface area against the external surface **23** of the catheter **20** and minimizes leaflet displacement during the process of staple or rivet fastening. An alternative example includes the use of auxiliary suctioning ports on the catheter 20 to assist in the grasping process; these may be independently translatable.

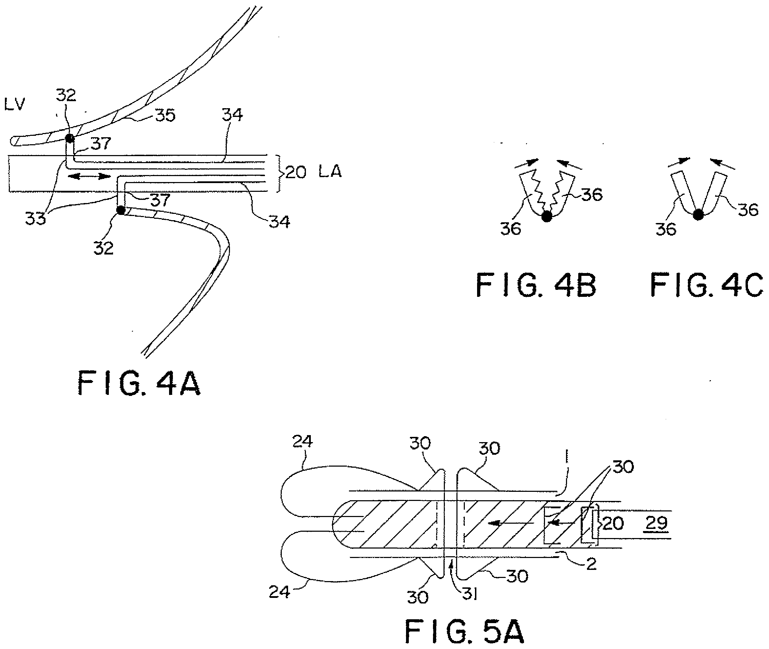

With reference to **Figure 4A****,** alternative example of the grasping elements are each comprised of a control rod **34** having a steerable distal end **33,** upon which is disposed a grabbing tool **32.** A variety of mechanisms for steering percutaneous surgical instruments are known to artisans and may be employed in steering the distal end **33** so as to position the grabbing tool **32** adjacent a portion of the atrial surface **35** to be grasped. Each distal end **33** is extended and retracted through a side port **37** or groove in catheter **20.** The grabbing tool **32,** in one example, has two pincer-like jaws **36** (two versions of which are shown in **Figures 4B, 4C**) that grasp the atrial surface **35** of the leaflet. One experienced in the art will recognize that a variety of other mechanisms currently available can be used as grabbing tools as an alternative to the jaws **36.** One such alternative includes the use of suction tips combined with pincers, a design serving to improve leaflet-catheter contact but not requiring as high a suction rate such as needed to grasp both leaflets simultaneously, the suction herein being used only for fine positioning and alignment.

In another example (not shown), more than one grasping element is employed to grasp the atrial surface of the leaflet. Multiple grasping elements extend in a similar direction away from the catheter to allow grasping of a selected axial length of the atrial surface. For example, there could be two grasping elements pointing to one side of the valve, and two to the other, thus stabilizing a square or rectangular portion of the leaflet surface.

In the embodiments described to this point, a single catheter **20** has been used to deliver and position the grasping elements to positions adjacent the ventricular or atrial surfaces of the leaflets. In a preferred embodiment, four independent grasping elements are used to grasp both surfaces of both leaflets, two elements per leaflet on opposite sides. This configuration provides the greatest flexibility in repositioning the leaflets into apposition and stabilizing them through the fastening step.

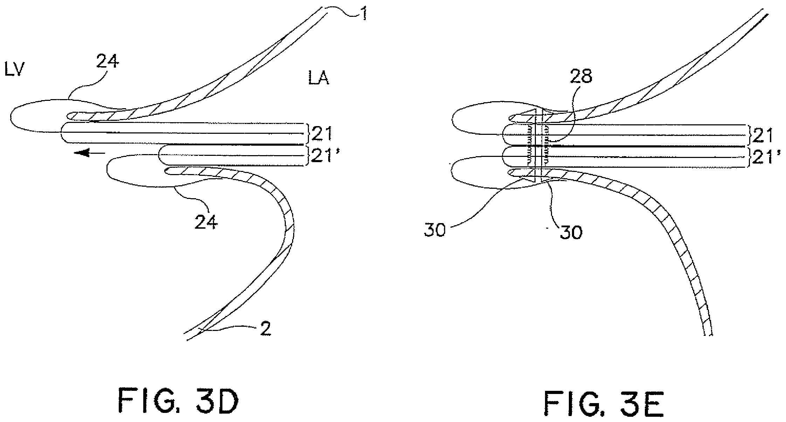

With reference to the embodiment in accordance with the invention shown in **Figures 3D** and **3E****,** the grasping elements **24** may be delivered independently through separate but linked catheters **21,21**'. The catheters **21,21'** are linked, for example, by a tongue and groove configuration, that allows them to move in a common plane. Translating the catheters achieves the desired leaflet **1,2** approximation after the grasping step. Each of the catheters **21,21'** in the catheter assembly has opening that together form a channel **28** through which the staple or rivet 30 is extended when the leaflets are repositioned in apposition.

As shown in **Figure 5A****,** once the leaflets **1,2** are grasped and approximated, one or more staples or rivets **30** are inserted through them. The staples or rivets **30** in one embodiment are composed of shape memory material and dimensioned to allow translation through the catheter **20** lumen and rapid expansion, effecting insertion and leaflet fastening, when the staples or rivets **30** reach an orifice **31** in the side wall of the catheter. The staples or rivets **30** are translated down the catheter **20** lumen by a plunger **29** until the orifice **31** is encountered. The staples or rivets **30** then emerge and pierce both leaflets, which have been previously stabilized by the grasping elements **24** described above. The shape memory staple or rivet emerges on the side of the leaflet opposite that of the catheter, and curves back to form a staple. Two or more such staples or rivets may be inserted at the same site, each facing in a different direction.

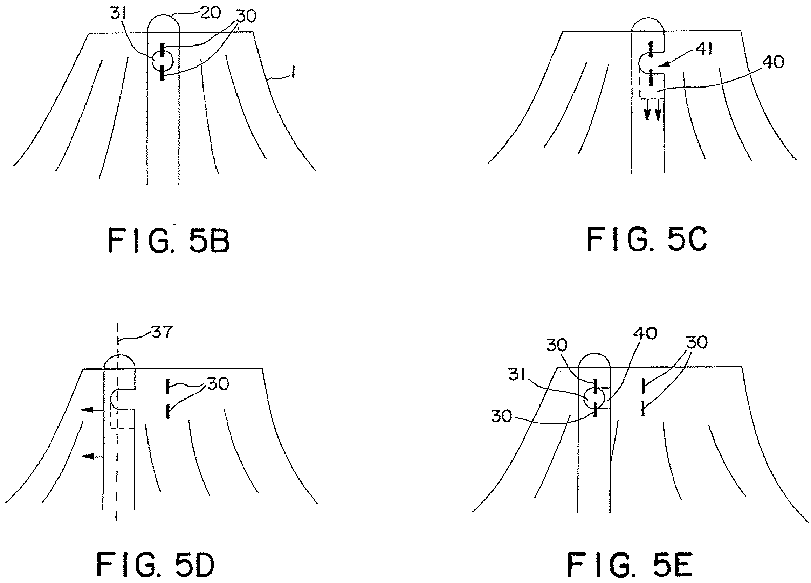

As shown in **Figure 5B****,** retractable door **40** in the side wall of the catheter can then be opened to disengage the catheter from the staples and move it to another site, so the leaflets can also be fastened together at additional locations to stabilize the repair, if necessary. After the catheter has been disengaged, the shape memory device continues to compress the leaflets together until an effective seal is formed.

**Figures 5B** through **5E** illustrate the sequence of maneuvers that achieve leaflet fastening at more than one site. In these figures, the cardiac valve is viewed from side to side as if looking down on its anterior leaflet **1** (as in the orientation shown in **Figure 3C**.) Catheter **20** is depicted as entering from an atrial route, although the same process can be practiced with the catheter entering retrograde from the aorta. In **Figure 5B****,** staples or rivets **30** have been inserted through the orifice **31** in the catheter that allows their extrusion into the leaflets. Turning to **Figure 5C****,** a portion **40** of the side wall of the catheter is retracted into the catheter lumen, activated by a wire or other connection to the catheter controls outside the patient. This step opens a hole **41** in the side of the catheter. The catheter is then disengaged from the staple or riveting device, and translated parallel to the catheter axis **37.** **Figure 5D** shows the catheter translated to a position closer to one side of the leaflet. **Figure 5E** shows the retractable catheter wall portion **40** replaced to re-form the piercing orifice **31** and allow fastening of the leaflets at another site. This can increase the efficacy of repair, as necessary, depending upon the precise geometry of the separated and regurgitant leaflets. This approach can be practiced with the linked sub-catheters shown in **Figures 3D** and **3E** as well, using retractable wall portions for each sub-catheter. Retractable portions of the catheter side walls may be placed on both sides of the catheter to allow translation in either direction. After one staple has been placed, with slight adjustment of the angle of catheter approach, the disengaged catheter 20 may also be translated parallel to its axis 37 to insert additional staples at more than one site along the central axis of the left ventricle, which is parallel to the initial catheter axis.

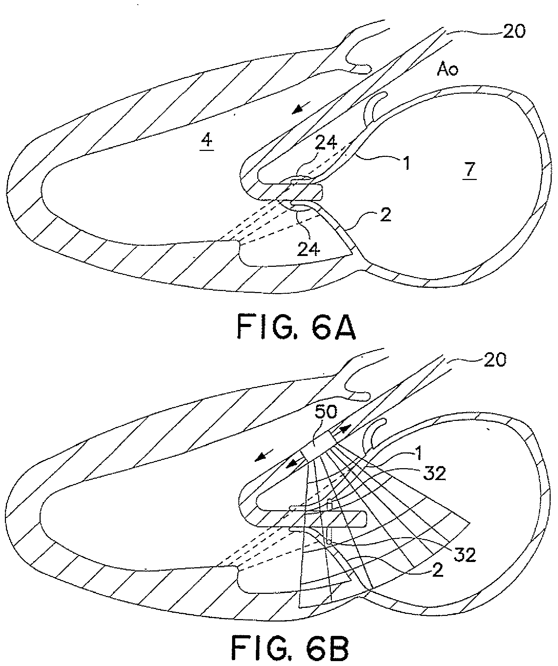

With reference to Figures 6A, and 6B, an alternative approach inserts the catheter 20 retrograde into the left ventricle 4 through the aorta. The catheter is shaped or steered to curve back toward the mitral leaflet tips using shape memory materials or other steering mechanisms known in the art. An advantage of the present invention is the need for only a single catheter assembly due to the novel leaflet grasping elements, as opposed to catheters coming from both aortic and artial routes. Leaflet-grasping elements, after positioning, are then applied to the distal leaflet tips. The grasping elements 24 are extended forward from the catheter onto the ventricular sides of the leaflet to surround the leaflet between catheter and clip. This forward grasping may be achieved through a number of mechanisms. For example, the grasping elements 24 may be comprised of preformed shape memory material that compresses the leaflets upon extension from side ports in the catheter. Alternatively, the grasping elements may be comprised of more rigid members that are hinged upon control rods within a sock-like component that, when extended beyond the hinge point, urges the members back towards the catheter, encompassing a portion of the leaflet therebetween.

With reference to Figure 6B, the entire process is preferably performed under the guidance of an imaging technique, such as echocardiography. Such guidance may be achieved using near infrared visualization (such as described in U.S. patent 6,178,346), with an intracardiac magnetic resonance imaging coil, or by transthoracic real-time three-dimensional echocardiographic imaging. Ultrasound imaging may be performed from the chest surface, esophagus, or within the heart, using an independent intracardiac echo (ICE) catheter (commercially available) within the adjacent right ventricular outflow tract, or an ultrasound
transducer within the left ventricle itself, built into the catheter 20. This arrangement allows an imaging element 50 to be maneuvered via the catheter to provide high-resolution images of the cardiac valve during the procedure, while also displaying the mitral leaflets and regurgitant jet. To produce a three-dimensional ultrasound image, a two-dimensional matrix away of piezoelectric crystals may be used, or a linear phased array may be rapidly rotated to produce a three-dimensional image.

The imaging may also be employed to confirm catheter contact with the leaflets and trigger the grasping step. The imaging element can be comprised of a simple piezoelectric crystal or optical coherence device that senses the motion of the leaflets. When the leaflet approaches the catheter in systole, the device outputs a triggering signal confirmable manually on an output display device. Once confirmed, the following triggering signal is output to a mechanism to translate the grasping element rapidly down the lumen to assume the retroflexing shape thereby effecting capture. In another embodiment, leaflet capture is effected by rapidly advancing a magnetic rod through the bore of the catheter to a position wherein the distal end of the extruded or extended grasping element, which in this embodiment is composed at least in part of a ferromagnetic material, is rapidly attracted toward the magnetic rod and the catheter. In yet another embodiment, a jointed rod is rapidly advanced so as to retroflex the grasping element against the leaflet.

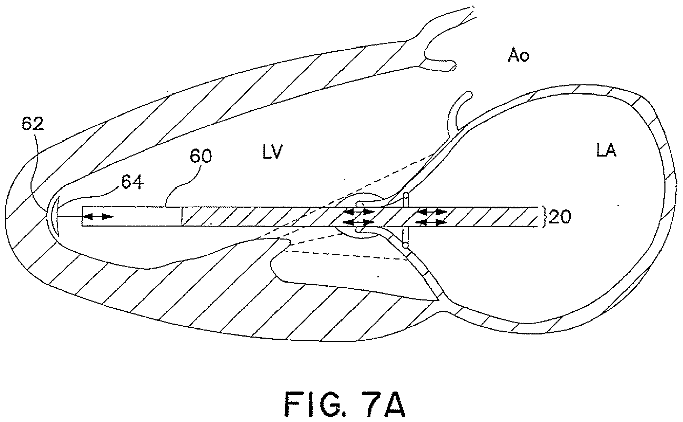

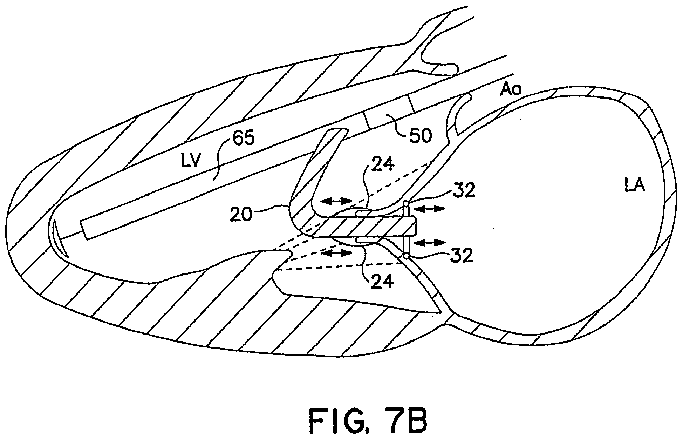

Both catheter introduction routes (through the mitral valve or the aorta) may also be practiced using a stabilizing catheter extension 60 advanced to the left ventricle apex 62, as shown in Figures 7A and 7B. From the extension 60 is extended a curving contact element 64 shaped to maintain contact with the apex 62. The contact element 64 may be made of shape memory elastic material which, when extruded from the catheter, forms a curving or tined contacting structure. Catheter 20 may then be translated along this wire to position the grasping elements 24,32 optimally in the region of the leaflet. For the transseptal route through the left atrium (Figure 7A), the stabilizing catheter extension 60 is in continuity with the fastening catheter 20, while for retrograde aortic routes (Figure 7B), the fastening catheter 20 emerges from an introducer catheter 65, and is steered toward the leaflets. The fastening catheter 20 is passed through a port in the introducer catheter 65, either directly or through a directing arm that may have an occlusive rubber seal. The introducer catheter 64 is stabilized by the curving contact element 64 extending beyond the catheter tip and shaped to maintain contact with the ventricular apex. The contact element 64 may alternatively be comprised of several elements composed of shape memory elastic material which, when extended from the catheter itself, form curving or tined contacting elements that may contact other portions of the interior of the cavity.

It is worth noting that the stabilizing mechanism described here is merely illustrative. The stabilizing mechanism is not limited to a wire or shape memory materials, nor is it limited to a single extendable contact element. For example, some embodiments may employ a plurality of stabilizing legs or telescoping extensions to contact inner left ventricle at numerous points around its circumference.

The device of this invention may also be practiced effectively in conjunction with procedures that assist in approximating the leaflets by inserting a mitral annular ring percutaneously. Such a device is inserted into the coronary sinus, a vein that encircles the mitral valve at the level of its annulus (the insertion of the leaflets onto the rest of the heart). Reducing annular size also helps in approximating the leaflets to achieve optimal repair. This combination of techniques utilizing the device of the present invention would provide a completely percutaneous approach to comprehensive repair of mitral valve prolapse and degenerative mitral regurgitation.

The present invention can also be used to treat ischemic mitral regurgitation in dilated hearts to reduce volume overload and prevent heart failure. Many of the more severe regurgitant lesions in such patients also involve malcoaption of non-opposed leaflet portions that can be repaired by this device. The present invention may be used with methods for reducing the tension on the leaflets to be so repaired, including percutaneous annular ring reductions and methods for reducing ventricular size. It can also be used to appose and link any two scallops of the posterior leaflet that may be malaligned or prolapse relative to each other, causing regurgitation.

## Claims

1. A device for repairing a cardiac valve, comprising:
a catheter assembly (20, 21, 21') configured to pass from a remote vasculature of a patient to a position within the heart adjacent the cardiac valve, wherein the catheter assembly (20, 21, 21') the catheter assembly being formed of two slidably linked catheters (21, 21');
two maneuverable grasping elements (24) each independently translatable through each catheter of the linked catheters, along a particular axis of extension of the catheter assembly, each of the grasping elements being extendable from a distal end of each catheter along an extension of direction to capture and reposition portions of two valve leaflets (1,2) into an apposed position, each of the grasping elements having a shape memory material with a pre-formed shape and including a tip that extends toward a proximal end of the catheter assembly in a relaxed state in which each grasping element extends from the distal end of each catheter so as to capture and reposition the respective portions at locations relative to the particular axis, the locations capable of being axially different from one another with respect to the particular axis; and
a fastening mechanism (30) translatable through the catheter assembly,
each catheter including at least one side port through which the fastening mechanism is extendable and disengageable to secure the repositioned portions of the valve leaflets.

2. The device according to claim 1, wherein the fastening mechanism is radially extendable and disengagable from the catheter assembly (20, 21, 21') through the at least one side port of the catheter assembly.

3. The device according to claim 2, wherein the fastening mechanism comprises a staple or rivet (30) insertable through each of the captured leaflet portions without displacing the leaflets (1, 2).

4. The device according to claim 2, wherein the fastening mechanism is composed at least in part of a shape memory material.

5. The device according to claim 2, further comprising a rectractable portion of the catheter assembly (20, 21, 21') proximate the at least one side port (37), in its retracted state creating a notch in the catheter assembly through which the fastening mechanism is disengagable from the catheter assembly.

6. The device according to claim 1, wherein each of the grasping elements extends from the distal end of the catheter assembly with reverse deformation, forming a reverse curve (25) in which the tip extends toward the proximal end of the catheter assembly with respect to the extension of direction so as to clip a ventricular surface (3) of one of the valve leaflets.

7. The device according to any of claims 1 - 6, further comprising an introducer catheter (65) for advancing the catheter assembly (20, 21, 21') toward the cardiac valve.

8. The device of claim 7, wherein the introducer catheter (65) further comprises
a directing arm through which the catheter assembly (20, 21, 21') that is maneuverable to a position proximate the cardiac valve.

9. The device of claim 7, wherein the introducer catheter (65) further comprises a first stabilization arrangement (32) configured to temporarily stabilize a position of the introducer catheter within the left ventricle.

10. The device of claim 9, wherein the introducer catheter (65) further comprises a second stabilization arrangement comprising one or more contact elements (64) reversibly extendable from the introducer catheter so as to contact an internal surface of the heart cavity at one or more points.

11. The device according to claim 8, wherein the one or more contact elements (69) are composed of shape memory elastic material.

12. The device according to any of claims 1 - 11, further comprising an imaging device (50) oriented so as to image a region near the cardiac valve.

## Patentansprüche

1. Vorrichtung zur Reparatur einer Herzklappe, aufweisend:
eine Katheteranordnung (20, 21, 21'), welche dazu eingerichtet ist, von einem entfernten Blutgefäß eines Patienten zu einer Position innerhalb des Herzens und benachbart zu der Herzklappe zu gelangen, wobei die Katheteranordnung (20, 21, 21') aus zwei verschiebbar verbundenen Kathetern (21, 21') geformt ist;
zwei bewegbar Greifelemente (24), von denen jedes unabhängig durch jeden der Katheter von den verbundenen Katheter entlang einer speziellen Erweiterungsachse der Katheteranordnung verschiebbar ist, wobei jedes der Greifelement von einem distalen Ende jedes Katheters entlang einer Richtungserstreckung erweiterbar ist, um Teilbereiche von zwei Klappenflügeln (1, 2) zu ergreifen und in eine gegenüberliegende Position neu zu positionieren,
wobei jedes der Greifelemente ein Formgedächtnismaterial mit einer vorgeformten Gestalt aufweisen und eine Spitze beinhalten, die sich in einem entspannten Zustand, in dem sich jedes der Greifelemente von dem distalen Ende jedes Katheters erstreckt, in Richtung auf ein proximales Ende der Katheteranordnung erstreckt, um die entsprechenden Teilbereiche an Stellen relativ zu der speziellen Achse zu ergreifen und neu zu positionieren, wobei die Stellen geeignet sind, in Bezug auf die spezielle Achse sich axial voneinander zu unterscheiden; und
einen Befestigungsmechanismus (30), welcher durch die Katheteranordnung verschiebbar ist,
wobei jeder Katheter wenigstens eine seitliche Öffnung beinhaltet, durch welche der Befestigungsmechanismus erweiterbar und lösbar ist, um die neu-positionierten Teilbereiche der Klappenflügel zu sichern.

2. Vorrichtung nach Anspruch 1,
wobei der Befestigungsmechanismus radial erweiterbar und lösbar von der Katheteranordnung durch die wenigstens eine seitliche Öffnung der Katheteranordnung ist.

3. Vorrichtung nach Anspruch 2,
wobei der Befestigungsmechanismus eine Klammer oder Niete (30) aufweist, welche durch jede der erfassten Flügelteile einführbar ist ohne die Flügel (1, 2) zu verschieben.

4. Vorrichtung nach Anspruch 2,
wobei der Befestigungsmechanismus zumindest teilweise aus einem Formgedächtnismaterial gebildet ist.

5. Vorrichtung nach Anspruch 2,
weiter aufweisend einen zurückziehbaren Teilbereich der Katheteranordnung (20, 21, 21'), der zu der wenigstens einen seitlichen Öffnung (37) benachbart ist, wobei diese in ihrem zurückziehbaren Zustand eine Kerbe in der Katheteranordnung bildet, durch welche der Befestigungsmechanismus von der Katheteranordnung lösbar ist.

6. Vorrichtung nach Anspruch 1,
wobei jedes der Greifelemente sich von dem distalen Ende der Katheteranordnung mit reverser Verformung erstreckt, wobei es eine reverse Kurve (25) formt, in welcher die Spitze sich in Richtung auf das proximale Ende der Katheteranordnung in Bezug auf die Richtungserstreckung erstreckt, um so eine ventrikuläre Oberfläche (3) von einer der Klappen festzuhalten.

7. Vorrichtung nach einem der Ansprüche 1 bis 6,
weiter aufweisend einen Einführungskatheter (65), um die Katheteranordnung (20, 21, 21') in Richtung auf die Herzklappe vorwärtszubewegen.

8. Vorrichtung nach Anspruch 7,
wobei der Einführungskatheter (65) weiterhin einen lenkenden Arm umfasst, durch den die Katheteranordnung (20, 21, 21') zu einer Position, die der Herzklappe benachbart ist, bewegbar ist.

9. Vorrichtung nach Anspruch 7,
wobei der Einführungskatheter (65) weiterhin eine erste Stabilisierungsanordnung umfasst, welche dazu eingerichtet ist, eine Position des Einführungskatheters (65) innerhalb des linken Ventrikels temporär zu stabilisieren.

10. Vorrichtung nach Anspruch 9,
wobei der Einführungskatheter (65) weiterhin ein oder mehrere Kontaktelemente umfasst, die von dem Einführungskatheter (65) revers ausfahrbarsind, um so eine innere Oberfläche des Herzraums an einem oder mehr Punkten zu kontaktieren.

11. Vorrichtung nach Anspruch 8,
wobei das eine oder die mehreren Kontaktelemente (69) aus einem Formgedächtniselastikmaterial gebildet sind.

12. Vorrichtung nach einem der Ansprüche 1 bis 11,
weiter aufweisend eine bildgebende Vorrichtung (50), welche so orientiert ist, dass sie einen Bereich in der Nähe der Herzklappe darstellt.

## Revendications

1. Un dispositif pour réparer une valve cardiaque, comprenant :
un ensemble formant cathéter (20, 21, 21') configuré pour passer d'un système vasculaire distant d'un patient à une position située dans le coeur, adjacente à la valve cardiaque, l'ensemble formant cathéter (20, 21, 21') étant formé de deux cathéters (21, 21') reliés de manière coulissante ;
deux éléments de préhension manoeuvrables (24) aptes chacun à être déplacés en translation indépendamment à travers chaque cathéter des cathéters reliés, selon un axe particulier d'extension de l'ensemble formant cathéter, chacun des éléments de préhension étant extensible depuis une extrémité distale de chaque cathéter selon une extension de direction pour saisir et repositionner des parties de deux valvules de valve (1, 2) vers une position apposée, chacun des éléments de préhension ayant un matériau à mémoire de forme ayant une forme préformée et comprenant une pointe qui s'étend vers une extrémité proximale de l'ensemble formant cathéter dans un état de relâchement dans lequel chaque élément de préhension s'étend depuis l'extrémité distale de chaque cathéter de manière à saisir et repositionner les parties respectives en des emplacements relatifs audit axe particulier, les emplacements pouvant être axialement différents l'un de l'autre par rapport à l'axe particulier ; et
un mécanisme de fixation (30) étant apte à être déplacé en translation à travers l'ensemble formant cathéter,
chaque cathéter comprenant au moins un orifice latéral par lequel le mécanisme de fixation est extensible et apte à être désengagé pour fixer les parties repositionnées des valvules de la valve.

2. Le dispositif selon la revendication 1, dans lequel le mécanisme de fixation est radialement extensible et apte à être désengagé de l'ensemble formant cathéter (20, 21, 21') au travers dudit au moins un orifice latéral de l'ensemble formant cathéter.

3. Le dispositif selon la revendication 2, dans lequel le mécanisme de fixation comprend une agrafe ou un rivet (30) apte à être inséré à travers chacune des parties de valvule saisies sans déplacer les valvules (1, 2).

4. Le dispositif selon la revendication 2, dans lequel le mécanisme de fixation est composé au moins en partie d'un matériau à mémoire de forme.

5. Le dispositif selon la revendication 2, comprenant en outre une partie rétractable de l'ensemble formant cathéter (20, 21, 21') à proximité dudit au moins un orifice latéral (37), dans son état rétracté, créant une encoche dans l'ensemble formant cathéter par laquelle le mécanisme de fixation est apte à être désengagé de l'ensemble formant cathéter.

6. Le dispositif selon la revendication 1, dans lequel chacun des éléments de préhension s'étend depuis l'extrémité distale de l'ensemble formant cathéter avec une déformation inverse, formant une courbe inverse (25) dans laquelle la pointe s'étend vers l'extrémité proximale de l'ensemble formant cathéter par rapport à l'extension de la direction afin de clipper une surface ventriculaire (3) de l'une des valvules de la valve.

7. Le dispositif selon l'une quelconque des revendications 1 à 6, comprenant en outre un cathéter d'introduction (65) pour faire avancer l'ensemble formant cathéter (20, 21, 21') vers la valve cardiaque.

8. Le dispositif selon la revendication 7, dans lequel le cathéter d'introduction (65) comprend en outre un bras directeur à travers lequel l'ensemble formant cathéter (20, 21, 21') qui est apte à être déplacé jusqu'à une position proche de la valve cardiaque.

9. Le dispositif selon la revendication 7, dans lequel le cathéter d'introduction (65) comprend en outre un premier dispositif de stabilisation (32) configuré pour stabiliser temporairement une position du cathéter d'introduction dans le ventricule gauche.

10. Le dispositif selon la revendication 9, dans lequel le cathéter d'introduction (65) comprend en outre un deuxième dispositif de stabilisation comprenant un ou plusieurs éléments de contact (64) extensibles de manière réversible à partir du cathéter d'introduction de manière à venir en contact avec une surface interne de la cavité cardiaque en un ou plusieurs points.

11. Le dispositif selon la revendication 8, dans lequel lesdits un ou plusieurs éléments de contact (69) sont composés d'un matériau élastique à mémoire de forme.

12. Le dispositif selon l'une quelconque des revendications 1 à 11, comprenant en outre un dispositif d'imagerie (50) orienté de manière à fournir une vue d'une zone située près de la valve cardiaque.
